# EUROPEAN PATENT SPECIFICATION

(11) **EP 2 701 789 B1**
(45) Date of publication and mention of the grant of the patent: **31.05.2017**
(21) Application number: 12777451.1
(22) Date of filing: 26.04.2012
(51) Int. Cl.: A61M 35/00, A61M 1/00, A61N 5/06, A61H 9/00, A61H 33/14, A61F 7/00, A61F 7/02

(54) **WOUND CARE SYSTEM WITH ONE OR BOTH OF VACUUM-LIGHT THERAPY AND THERMALLY AUGMENTED OXYGENATION**
WUNDPFLEGESYSTEM MIT EINER VAKUUMLICHTTHERAPIE UND/ODER WÄRMEVERSTÄRKTER SAUERSTOFFANREICHERUNG
SYSTÈME DE TRAITEMENT DES PLAIES PAR PHOTOTHÉRAPIE SOUS VIDE ET/OU OXYGÉNATION THERMIQUEMENT AUGMENTÉE

(30) Priority: 26.04.2011 US 201161479156 P; 26.01.2012 US 201213359210
(43) Date of publication of application: 05.03.2014
(60) Divisional application: 17163036.1
(73) Proprietor: Thermotek, Inc., Flower Mound, TX 75028 (US)
(72) Inventor: QUISENBERRY, Tony, Highland Village TX 75077 (US)
(74) Representative: Protector IP Consultants AS
(86) International application number: PCT/US2012/035096
(87) International publication number: WO 2012/149092

(56) References cited:
- EP-A1- 0 076 074
- WO-A1-2010/124234
- US-A- 4 471 787
- US-A- 4 926 881
- US-A- 4 995 698
- US-A1- 2006 282 028
- US-A1- 2007 032 778
- US-A1- 2008 071 330
- US-A1- 2008 071 330
- US-A1- 2010 150 991
- US-A1- 2011 034 861
- US-B1- 7 427 153

## Description

### BACKGROUND OF THE INVENTION

### Technical Field

The present invention relates to a combination therapy pad for use in wound care method and system with one or both of vacuum-light therapy, pulsed radio frequency ("RF"), and thermally augmented oxygenation

### Description of the Related Art

An important aspect of patient treatment is wound care. Medical facilities are constantly in need of advanced technology for the cleaning and treatment of skin wounds. The larger the skin wound, the more serious the issues are of wound closure and infection prevention. The rapidity of the migration over the wound of epithelial and subcutaneous tissue adjacent the wound is thus critical. Devices have been developed and/or technically described which address certain aspects of such wound healing. For example, U.S. Patent No. 6,695,823 to Lina et al. ("Lina") describes a wound therapy device that facilitates wound closure. A vacuum pump is taught for collecting fluids from the wound. WO 93/09727 discloses a solution for wound drainage by utilizing negative pressure over the wound to promote the above references migration of epithelial and subcutaneous tissue over the wound.

In other embodiments, wound treatment is performed using light therapy. For example, U.S. Patent No. 7,081,128 to Hart et al. ("Hart") describes a method of treating various medical conditions such as, for example, joint inflammation, edema, etc., utilizing an array of Light Emitting Diodes contained on a flexible substrate that may be wrapped around an anatomical feature of the human body. US Patent No. 6,596,016 to Vreman et al. ("Vreman") discloses a phototherapy garment for an infant having a flexible backing material, a transparent liner, and a flexible printed circuit sheet containing surface-mounted LEDs. The LEDs preferably emit high-intensity blue light, suitable for the treatment of neonatal hyperbilirubinemia. The device may include a portable power supply.

In other embodiments, wound treatment is performed using oxygen. The use of oxygen for the treatment of skin wounds has been determined to be very beneficial in certain medical instances. The advantages are multitudinous and include rapidity in healing. For this reason, systems have been designed for supplying high concentration of oxygen to wound sites to facilitate the healing process. For example, U.S. Patent No. 5,578,022 to Scherson et al. ("Scherson") teaches an oxygen producing bandage and method. One of the benefits cited in Scherson is the ability to modulate a supply of concentrated hyperbaric oxygen to skin wounds. Although oxygen is beneficial in direct application of predetermined dosages to skin wounds, too much oxygen can be problematic. Oxygen applied to a wound site can induce the growth of blood vessels for stimulating the growth of new skin. Too much oxygen, however, can lead to toxic effects and the cessation of healing of the wound. It would be an advantage, therefore, to maximize the effectiveness of oxygen applied to a wound area by enhancing the absorption rate of oxygen into the skin and tissue fluids. By enhancing the absorption rate of the oxygen in the wound, less exposure time and concomitantly fewer toxic side effects to the endothelial cells surrounding the wound, such as devasculation, occurs. It would be a further advantage, therefore, to utilize existing medical treatment modalities directed toward other aspects of patient therapy to augment oxygenation for wound care.

It has been accepted for many years by medical care providers that patient thermal therapy can be very advantageous for certain injuries and/or post operative recovery. For this reason, thermal therapy has been advanced and many reliable and efficient systems exist today which provide localized thermal therapy to patients in both pre and post surgical environments. In particular, absorption of oxygen by cells is enhanced by contrast thermal therapy wherein the wound area is heated prior to being saturated with oxygen and subsequently cooled.

Addressing first thermal therapy systems, several devices have been engineered to deliver temperature controlled fluids through pads or convective thermal blankets to achieve the above purpose. Typically, these devices have a heating or a cooling element, a source for the fluid, a pump for forcing the fluid through the pad or blanket, and a thermal interface between the patient and the temperature controlled fluid. U.S. Patent No. 4,884,304 to Elkins ("Elkins") is, for example, directed to a mattress cover device which contains liquid flow channels which provide the selective heating or cooling by conduction.

Further, US 2006/282028 A1, to Howard et al, discloses a wound therapy pad system with three attached layers comprising a fiber-optic array in e.g. the top and bottom layers or in the middle layer. The middle layer further comprises tubing connected to a vacuum source and in fluid communication with a therapeutic fluid source.

US 2008/071330 A1 to Quisenberry et al, discloses a therapy system comprising a combination therapy pad used to administer vacuum, thermal and light therapy.

Devices have also been developed for simply providing heat or cooling to a person in bed. Electric blankets containing electric heating elements have been used, for example, to provide heat to people in bed. Likewise, cooling blankets, such as the blanket disclosed in U.S. Patent No. 4,660,388 to Greene ("Greene"), have also been proposed. Greene discloses a cooling cover having an inflatable pad with plenum chambers at opposite ends thereof. Cool air is generated in a separate unit and directed to the pad and out to a number of apertures on the underside of the pad and against the body of the person using the cover.

A disposable heating or cooling blanket is disclosed in U.S. Patent No. 5,125,238 to Ragan et al. ("Ragan"), which has three layers of flexible sheeting. Two of the layers form an air chamber while a third layer includes a comfortable layer for contact with the patient. Conditioned air is directed toward the covered person through a multiplicity of orifices in the bottom layers of the blanket.

A temperature controlled blanket and bedding assembly is also disclosed in U.S. Patent No. 5,989,285 to DeVilbiss et al. ("DeVilbiss"), assigned to the assignee of the present invention. DeVilbiss discloses a temperature controlled blanket and temperature control bedding system having the provision of both recirculating temperature controlled fluid and temperature controlled gas to enhance performance for convectively heating or cooling a patient. Counterflow or co-flow heat exchanging principles between the temperature controlled liquid and the temperature controlled gas achieve temperature uniformity across different sections of the blanket and the bedding system. Drapes and the temperature controlled bedding system provide a temperature controlled envelope around a person using the bedding system. In one embodiment of the bedding system, the air portion of the bedding system is provided for use with a patient that supplies the fluid portion of the overall bedding system. In another embodiment of the bedding system, the fluid portion of the bedding system is provided for use with a patient bed which supplies the air portion of the overall bedding system.

U.S. Patent No. 5,097,829 to Quisenberry ("Quisenberry") describes an improved temperature controlled fluid circulating system for automatically cooling a temperature controlled fluid in a thermal blanket with a thermoelectric cooling device having a cold side and a hot side when powered by electricity. The temperature controlled fluid is cooled by the cold side of the cooling device and pumped through, to, and from the blanket through first and second conduits.

Finally, co-pending U.S. Patent Application No. 10/894,369, assigned to the assignee of the present invention, teaches a sequential compression blanket for use with heating or cooling therapy. In this particular embodiment, the utilization of thermal therapy with sequential compression in a programmable format which further has the option of the introduction of oxygenation through a perforated membrane disposed between the patient and the thermal therapy pad is taught. These advances in the medical industry have been recognized as advantageous to both the medical care providers as well as the patients. The precise manner of oxygenation application is, however, still in the process of development.

The present invention provides improvements in wound care by providing multiple wound healing approaches such as, for example, the application of negative pressure over the wound area along with light therapy of the wound area, and oxygenation of the wound area in conjunction with thermal therapy. By combining an oxygenation modality that is utilized in conjunction with light and thermal therapy and/or sequential compression in association therewith, the individual benefits of negative wound pressure, light therapy, and oxygenation treatments can be synergistically enhanced.

### SUMMARY

.

Present invention relates to a combination therapy pad that includes a first layer and a second layer operatively coupled to the first layer. A fiber-optic array is disposed between the first layer and the second layer. A third layer is operatively coupled to the first layer. The third layer includes a vacuum tube in fluid communication with a vacuum source and a therapeutic fluid tube in fluid communication with a therapeutic fluid source. The third layer provides at least one of vacuum therapy and therapeutic fluid treatment to a wound area. The first layer comprises a first central gap and the second layer comprises a second central gap substantially aligned with the first central gap, and at least a portion of the fiber-optic array is disposed in the first central gap and the second central gap.

According to a preferred embodiment, the portion of the fiber-optic array is preferably placed in direct contact with the wound area. The second layer preferably comprises an adhesive bottom surface.

Further, the combination therapy pad can include a recessed central area in fluid communication with the vacuum tube and the therapeutic fluid tube is preferably defined within the third layer. An antenna is preferably disposed substantially around a perimeter of the recessed central area, wherein the antenna receives pulsed radio-frequency signals.

In yet another preferred embodiment, the first layer, the second layer and the third layer are joined via at least one of adhesive bonding and welding.

### BRIEF DESCRIPTION OF THE DRAWINGS

A more complete understanding of the combination therapy pad of the present invention may be obtained by reference to the following Detailed Description when taken in conjunction with the accompanying Drawings wherein:
FIGURE 1 is an illustration of a wound care system;
FIGURE 2 is a block diagram;
FIGURE 3 is a flow diagram of a process;
FIGURE 4 illustrates a side elevational cross sectional view of a therapy blanket/pad according to an exemplary embodiment;
FIGURE 5 illustrates a side elevational cross sectional view of a therapy blanket/pad according to an exemplary embodiment;
FIGURE 6 is a diagrammatic illustration of a therapy blanket/pad according to an exemplary embodiment;
FIGURE 7 is a diagrammatic illustration of a wound evacuation and UV LED treatment pad according to an exemplary embodiment;
FIGURE 8A is a schematic diagram of a wound care system;
FIGURE 8B is a front perspective view of a wound care system;
FIGURE 8C is a front perspective view of a wound care system illustrating a plurality of hooks;
FIGURE 9 is a is a block diagram of a wound care system;
FIGURE 10 is a block diagram of a wound care system;
FIGURE 11 is a diagrammatic illustration of a combination therapy pad according to an exemplary embodiment;
FIGURE 12 is a diagrammatic illustration of a combination therapy pad according to an exemplary embodiment; and
FIGURE 13 is an exploded view of a combination therapy pad according to an exemplary embodiment.

### DETAILED DESCRIPTION

Various embodiments of the present invention will now be described more fully with reference to the accompanying drawings. The invention may, however, be embodied in many different forms and should not be construed as limited to the embodiments set forth herein; rather, the embodiments are provided so that this disclosure will be thorough and complete, and will fully convey the scope of the invention to those skilled in the art.

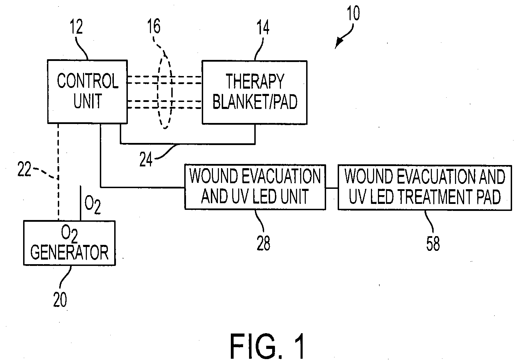

Referring first to FIGURE 1, there is shown an illustration of one embodiment of a wound care system 10. The system 10 comprises a control unit 12, a therapy blanket/pad 14 and a plurality of tubular members 16 (to be defined below) connecting the control unit 12 to the therapy blanket/pad 14. The system 10 further includes a wound evacuation and ultra violet light emitting diode (UV LED) unit 28 and a wound evacuation and UV LED treatment pad 58. The wound evacuation and UV LED unit 28 is connected to the control unit 12 while the wound evacuation and UV LED treatment pad 58 is connected to the wound evacuation and UV LED unit 28. A system for providing both oxygenation therapy in conjunction with certain aspects of thermal therapy and fully describing the thermal operation and sequence compression aspects is set forth and shown in U.S. Patent Application No 10/894,369, assigned to the assignee of the present invention. For that reason, thermal detail relative to the interaction between the control unit 12 and the therapy blanket/pad 14 relative to the thermal fluid flow and pressurization for sequenced compression therapy is not further defined herein. What is defined, is the added aspect of wound care provided by wound evacuation and light therapy. Light therapy is the application of light energy to the skin for therapeutic benefits. LED light therapy promotes wound healing and human tissue growth. Energy delivered by the LEDs enhances cellular metabolism, accelerates the repair and replenishment of damaged skin cells, as well as stimulates the production of collagen which is the foundation of a healthy and smooth skin. Light therapy is non-ablative, non-invasive, and painless.

Still referring to FIGURE 1, the use of the therapy blanket/pad 14 to the wound site of the patient may be, in one embodiment, subsequent to the cleaning of the wound area of dead tissue by the wound evacuation and UV LED treatment pad 58. Velcro cross straps may be utilized to secure the therapy blanket/pad 14. A 93% concentration of oxygen has been suggested to be advantageous when applied to a wound site as described herein with one or two atmospheres of pressure. An oxygen generator/concentrator 20 may be utilized within the control unit 12 or may be separate therefrom. In FIGURE 1, an oxygen generator/ concentrator 20 is shown in association with the control unit 12 by dotted line 22 and an oxygenation gas line 24 shown extending between the control unit 12 and the therapy blanket/pad 14..

In FIGURE 1, fiber optic strands (not explicitly shown) direct ultraviolet light from a plurality of LEDs (not explicitly shown) to an array of fiber optic strand ends (not explicitly shown) located on the undersurface of wound evacuation and UV LED treatment pad 58. The control unit 12 may be used to modulate the ultraviolet light to create various patterns of light, different intensities of light, and different durations of light. For example, the control unit 12 may be used to generate pulsed emission of ultraviolet light. The ultraviolet light is capable of penetrating through several layers of skin to destroy infectious bacteria. In one embodiment, not specifically shown herein, the UV LED treatment pad 58 may be provided on the therapy blanket/pad 14. According to exemplary embodiments, the ultraviolet light from the plurality of LEDs located on the undersurface of wound evacuation and UV LED treatment pad 58 destroys a wide variety of microorganisms such as, for example, bacteria which causes skin infections. In addition, the ultraviolet light from the plurality of LEDs improves wound healing along with cell and bone growth. Furthermore, the use of LEDs in light therapy is safe, non-invasive, drug-free and therapeutic.

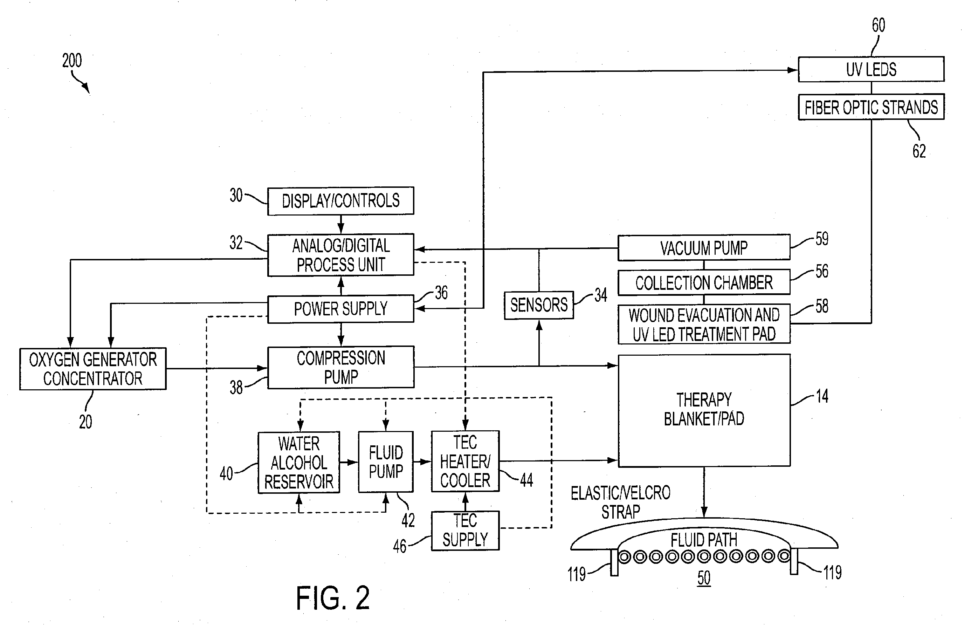

Referring now to FIGURE 2, there is a block diagram 200 illustrating the flow of oxygenation gas as a transfer fluid. As set forth in the block diagram 200, a control unit display 30 is provided in conjunction with an analog/digital processing unit 32. A plurality of sensors 34 are utilized in conjunction with the processing unit 32 for control of heat transfer fluids to the therapy blanket/pad 14 as well as the oxygen delivery thereto. The oxygen generator/concentrator 20 is connected to a power supply 36, which power supply 36, also powers the processing unit 32. The oxygen generated from the oxygen generator/concentrator 20 is then pumped through compression pump 38 before delivery to the therapy blanket/pad 14. It should be noted that an oxygen supply may also be used.

Referring still to FIGURE 2, a water/alcohol reservoir 40 is shown in fluid flow communication with fluid pump 42 and Thermo Electric Cooler (TEC) heater/cooler 44. The TEC heater/cooler 44 is controlled by the processing unit 32 and a TEC supply 46 is likewise shown. Adjacent the TEC supply 46 is illustrated a diagrammatical schematic of a treatment chamber 50 defined beneath the therapy blanket/pad 14 wherein the treatment chamber 50 is thermally exposed to the thermal fluid by the fluid path therein illustrated. The adhesive attachment edges 52 therein shown likewise define the treatment chamber space 50 between the therapy blanket/pad 14 and the wound site to allow for the flow of the oxygenation gas therein.

Referring still to FIGURE 2, there is shown a vacuum pump 59 powered by the power supply 36. A collection chamber 56 is connected to the vacuum pump 59 and to a wound evacuation and UV LED treatment pad 58. The wound evacuation and UV LED treatment pad 58 is used prior to the therapy blanket/pad 14 for cleaning the wound area in preparation for oxygenation in conjunction with thermal therapy. Referring still to FIGURE 2, there is shown a plurality of ultraviolet LEDs 60 and fiber optic strands 62, which are interoperably connected to the wound evacuation and UV LED treatment pad 58. The wound evacuation and UV LED treatment pad 58 is used prior to the therapy blanket/pad 14, in one embodiment, for removing bacteria from the wound area in preparation for oxygenation in conjunction with thermal therapy in accordance with an embodiment. Ultraviolet light from the plurality of LEDs 60 destroys a wide variety of microorganisms such as, for example, bacteria which causes skin infections. In addition, the ultraviolet light from the plurality of LEDs 60 improves wound healing along with cell and bone growth. Furthermore, the use of the plurality of LEDs 60 in light therapy is safe, non-invasive, drug-free and therapeutic.

The ultraviolet light from the plurality of LEDs 60 is in the range of approximately 200 to 450 nanometers and higher, and energy levels of up to 35,000 microwatt seconds/cm², which are necessary to eliminate or destroy most microorganisms such as bacteria, spores, algae and viruses. Most bacteria can be destroyed at ultra violet energies of from about 3,000 to about 5,000 microwatt-seconds/cm² while mold spores may require energies in the 20,000 to 35,000 mW-seconds/cm².

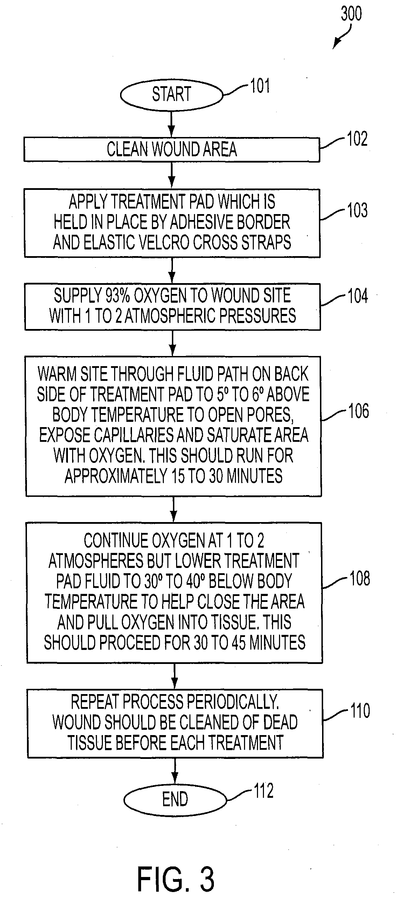

Referring now to FIGURE 3 there is shown a flow diagram of a process 300. The process 300 starts at step 101. At step 102, the wound area is cleaned of dead tissue, any undesirable fluids, and bacteria by applying the wound evacuation and UV LED treatment pad 58. The wound evacuation and UV LED treatment pad 58 is used prior to the therapy blanket/pad 14 for removing bacteria from the wound area in preparation for oxygenation in conjunction with thermal therapy. According to exemplary embodiments, the ultraviolet light from the plurality of LEDs located on the undersurface of wound evacuation and UV LED treatment pad 58 destroys a wide variety of microorganisms such as, for example, bacteria which causes skin infections. In addition, the ultraviolet light from the plurality of LEDs improves wound healing along with cell and bone growth. Furthermore, the use of LEDs in light therapy is safe, non-invasive, drug-free and therapeutic.

At step 103, the therapy blanket/pad 14 is applied to the wound area. The therapy blanket/pad 14 is held in position by an adhesive border and, in one embodiment, elastic Velcro cross straps. At step 104, according to an embodiment, an oxygenation gas comprising on the order of 93% concentration of oxygen gas is delivered to the wound site with one to two atmospheric pressures. The numbers as set forth and shown are exemplary and other oxygenation concentrations as well as pressures are contemplated. Consistent therewith, however, is the concept of, and teachings for, thermal treatment of the wound site in conjunction with oxygenation. In step 106, the site is warmed through the fluid path herein shown on the back side of the therapy blanket/pad 14 up to approximately 5 to approximately 6 degrees above the body temperature of the patient. Warming allows the pores of the patient's skin to open, exposing capillaries therein. The capillaries of the skin are then saturated with oxygen. In one period of time herein described, a warming period of approximately 15 to approximately 30 minutes is recommended. At step 108, oxygenation is continued at one to two atmospheres and the therapy blanket/pad fluid is lowered to approximately 30 to approximately 40 degrees below body temperatures. Cooling closes the pores of the wound area and pulls oxygen into the underlying tissue. Cooling then proceeds for approximately 30 to approximately 45 minutes in accordance with an embodiment. At step 110, the process 300 may be repeated periodically and the wound area may be cleaned of dead tissue before each treatment. At step 112, the process 300 ends.

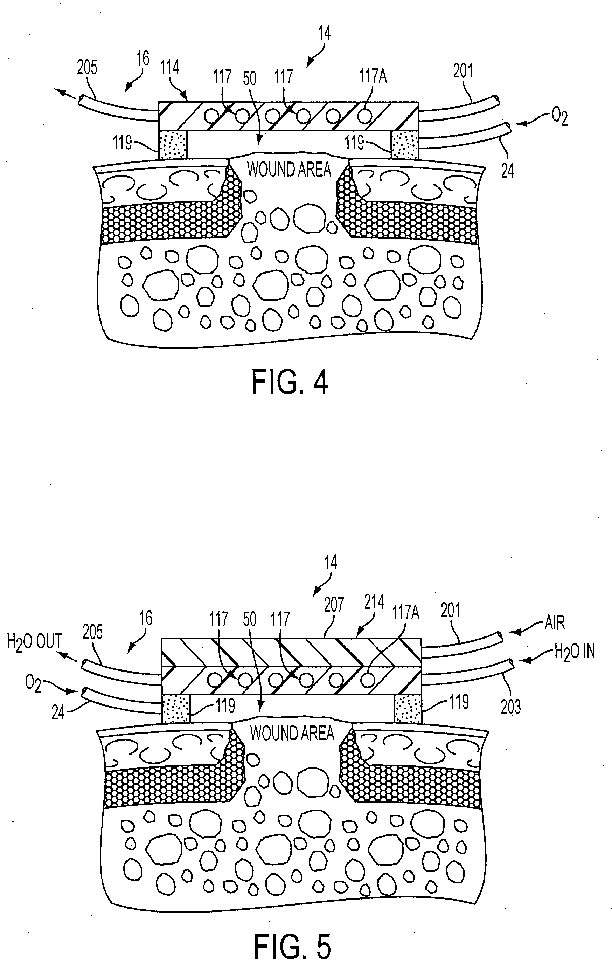

FIGURE 4 is a side elevational, cross sectional view of one embodiment of the therapy blanket/pad 14. In an embodiment, the therapy blanket/pad 14 is constructed with a single bladder 114 where thermal fluid flow may be provided. The tubular members 16 are coupled to the therapy blanket/pad 14. The therapy blanket/pad is fabricated with a circuitous flow path therein for thermal fluid flow. The circuitous flow path may be tubular in form, or simply a path within therapy blanket/pad 14 defined by flow channels. What is shown is a path 117 within therapy blanket/pad 14. The path 117 is shown with tubular ends 117A, for example, illustrating that thermal fluid flows therein for thermal treatment of the underlying wound area. Again, the path 117 may not be of tubular form and may have a variety of shapes and fabrication techniques well known in the art of thermal pads.

According to an exemplary embodiment, the therapy blanket/pad 14 is separated from the patient's skin by adhesive strips 119 having a thickness of, for example, 1/8 inch. The therapy blanket/pad 14 (not drawn to scale) exposes the wound to both heat and cold via the path 117 while oxygen is injected into the treatment chamber 50. The injection of oxygen in conjunction with the aforesaid heating and cooling via the path 117 helps treat the wound area and any stasis zones therein where tissue swelling has restricted flow of blood to tissues within the wound area. It is well known that, without sufficient blood flow, the epithelial and subcutaneous tissues referenced above receive less oxygen and are less able to migrate over the wound area to promote healing. By utilizing the embodiments disclosed herein, oxygenation is enhanced and the problems associated with such conditions are mitigated.

FIGURE 5 illustrates an exemplary embodiment of the thermal therapy and oxygenation treatment pad of FIGURE 4. A dual bladder 214 is thus provided where air may be applied to second bladder 207 atop the path 117, also represented by the "tubular" ends 117A shown for purposes of example only. In this manner, select compression therapy may be afforded in conjunction with the thermal and oxygenation treatment. In that regard, air inlet tube 201 is connected to the second bladder 207. Both FIGURES 4 and 5 show oxygen tube 24 for feeding oxygen to the treatment chamber 50, with tube 203 allowing thermal fluid into conduits 117 with tube 205 allowing thermal fluid return to control unit 12 of FIGURE 1. FIGURE 5 further illustrates the advantages of FIGURE 4 with the ability for either compression or sequenced compression as referenced above.

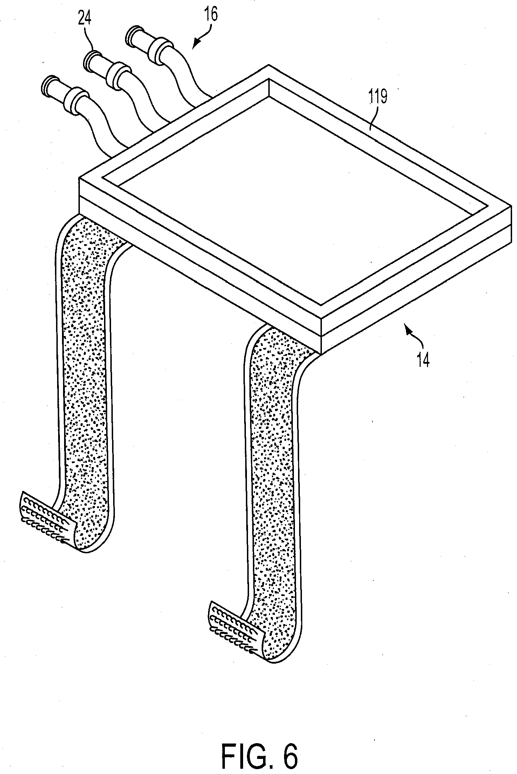

Referring now to FIGURE 6, there is shown a diagrammatic illustration of the therapy blanket/pad 14 of FIGURES 1 and 4. The tubular members 16 for thermal fluid flow and the tube 24 for oxygen flow are clearly seen. The adhesive border 119 is likewise shown.

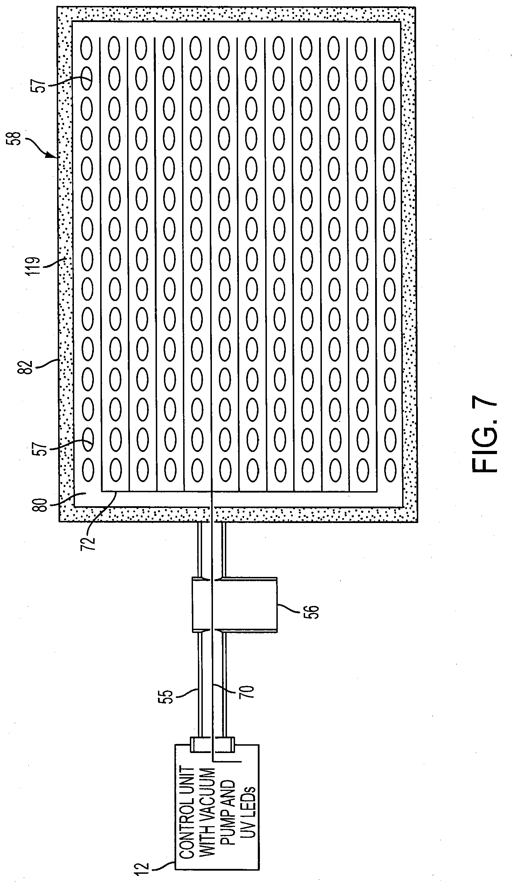

FIGURE 7 is diagrammatic illustration of a wound evacuation and UV LED treatment pad 58 according to an embodiment of the present invention. In this embodiment, the wound evacuation and UV LED treatment pad 58 contains an array of fiber optic strand 72 to project ultraviolet light onto a wound area (not explicitly shown). In a typical embodiment, the fiber optic strands 72 may be cleaved side emitting fibers. The wound evacuation and UV LED treatment pad 58 also contains an array of unique removal ports 57 that may be used to remove any undesirable fluid from the wound area. The wound evacuation and UV LED treatment pad 58 further contains a non-tissue adhesive service 80 which contains both the fiber optic strand array 72 and the unique removal ports 57. An adhesive circumference 82 is located around the periphery of the wound evacuation and UV LED treatment pad 58 to allow for a seal to be formed around the wound area. The seal, in conjunction with the removal ports 57, allows a negative pressure to form over the wound area. Negative pressure facilitates removal undesirable tissues from the wound area. The wound evacuation and UV LED treatment pad 58 is connected to a control unit 12. The control unit 12 contains a vacuum pump (not shown) and a plurality of ultraviolet LEDs (not explicitly shown). The vacuum pump is connected to the wound evacuation and UV LED treatment pad 58 via a vacuum line 55. A collection chamber 56 is positioned between the vacuum pump and the wound evacuation and UV LED treatment pad 58 to intercept and store undesirable fluids, tissues, and the like that are removed from the wound area as a result of negative pressure applied to the wound area with the vacuum pump. The plurality of ultraviolet LEDs transmit ultraviolet light through the fiber optic strands 70 to the wound evacuation and UV LED treatment pad 58, where the fiber optic strands 70 are then dispersed throughout the wound evacuation and UV LED treatment pad 58 to project ultraviolet light onto the wound area. Energy delivered by the plurality of LEDs enhances cellular metabolism, accelerates repair and replenishment of damaged skin cells, as well as stimulates production of collagen which is the foundation of a healthy and smooth skin. Light therapy is non-ablative, non-invasive, and painless.

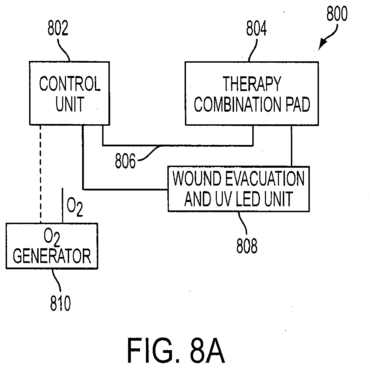

FIGURE 8A is a schematic diagram of a wound care system according to an exemplary embodiment. A wound care system 800 includes a control unit 802, a combination therapy pad 804, and a plurality of tubular members 806 connecting the combination therapy pad 804 to the control unit 802. A wound evacuation and UV-LED unit 808 is associated with the control unit 802 and connected to the combination therapy pad 804. In various embodiments, the wound evacuation and UV-LED unit 808 and the control unit 802 are contained in a single housing; however, in various alternative embodiments, the wound evacuation and UV-LED unit 808 and the control unit 802 may not be in a single housing and are independent devices.

Still referring to FIGURE 8A, use of the combination therapy pad 804 incorporates ultraviolet light and evacuation therapy for wound cleaning with thermal and oxygenation therapy known to promote healing. In various embodiments, Velcro cross straps are used to secure the combination therapy pad 804. An oxygen generator/concentrator 810 is utilized to provide, for example, a 93% concentration of oxygen to a wound site via the combination therapy pad 804. In a typical embodiment, the oxygen generator/concentrator 810 and the control unit 802 are separate devices; however, in other embodiments, the oxygen generator/concentrator 810 and the control unit 802 are contained in a single housing.

Still referring to FIGURE 8A, fiber optic strands (not explicitly shown) direct ultraviolet light from a plurality of LEDs (not explicitly shown) located in the wound evacuation and UV-LED unit 808 to an array of fiber optic strands (not explicitly shown) located on an undersurface of the combination therapy pad 804. The control unit 802 may be used to modulate the ultraviolet light to create, for example, various patterns of light, different intensities of light, and different durations of light. For example, in various embodiments, the control unit 802 is used to produce pulsed emission of the ultraviolet light.

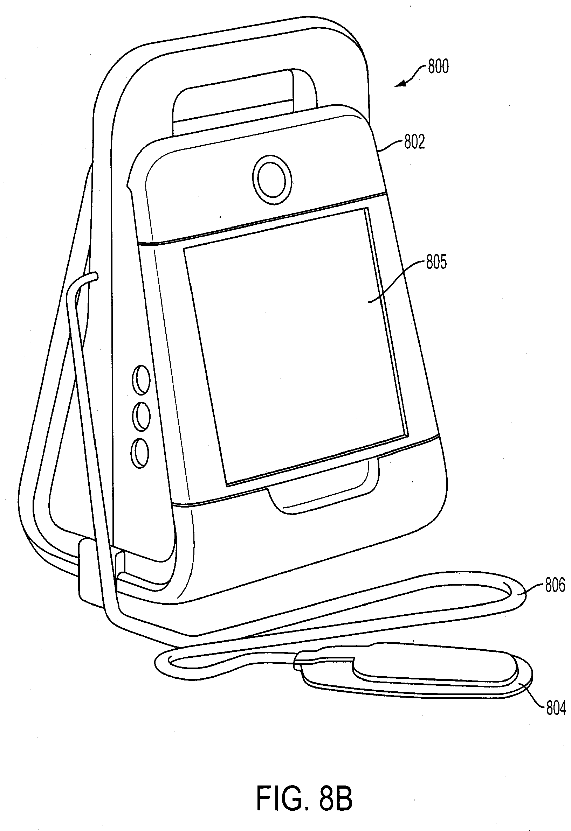

FIGURE 8B is a front perspective view of a wound care system according to an exemplary embodiment. The wound care system 800 includes the control unit 802, the combination therapy pad 804, and the plurality of tubular members 806 connecting the combination therapy pad 804 to the control unit 802. A user interface 805 is disposed on a front surface of the control unit 802. In a typical embodiment, the user interface 805 allows a user to control various parameters of wound care-treatment including, for example, oxygen concentration, oxygen pressure, temperature, and ultra-violet light intensity. The user interface 805 may be pivoted relative to the control unit 802 to provide a favorable viewing angle. In a typical embodiment, the user interface 805 may be, for example a touch screen interface; however, in other embodiments, the user interface 805 may be, for example, a plurality of controls or any other user interface. Use of the combination therapy pad 804 incorporates ultraviolet light and evacuation therapies for wound cleaning with thermal and oxygenation therapy known to promote healing. In various embodiments, Velcro cross straps (not shown) may be used to secure the combination therapy pad 804.

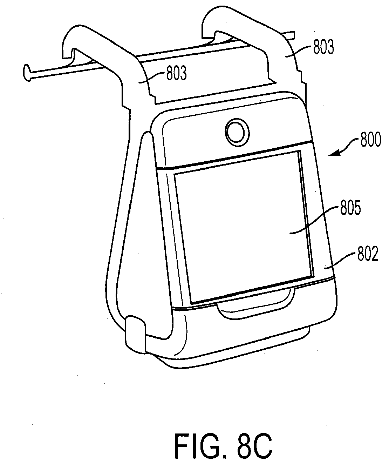

FIGURE 8C is a front perspective view of the wound care system of FIGURE 8A illustrating a plurality of foldable hooks. The wound care system 800 includes a plurality of foldable hooks 803 disposed, for example, along a top of the control unit 802. In a typical embodiment, the plurality of foldable hooks 803 may be utilized to hang the control unit 802 from, for example, a hospital bed.

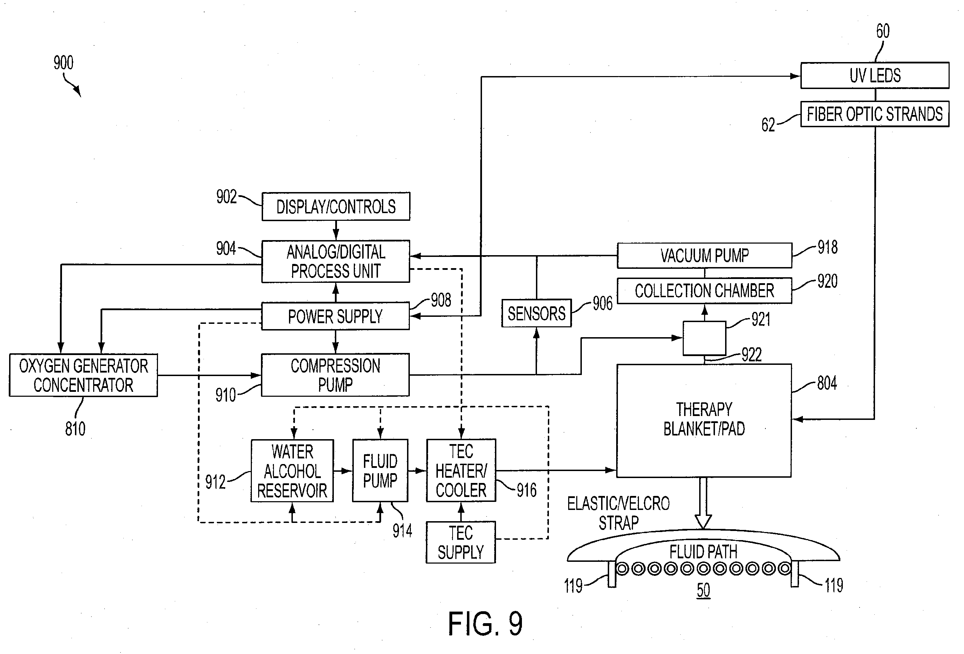

FIGURE 9 is a block diagram of a wound care system according to an exemplary embodiment. In a wound-care system 900, a control unit display 902 is provided in conjunction with a processing unit 904. In a typical embodiment, the processing unit 904 is an analog/digital processing unit. A plurality of sensors 906 are utilized in conjunction with the processing unit 904 for control of heat transfer fluids to a combination therapy pad 804. In various embodiments, the oxygen generator/concentrator 810 is connected to a power supply 908. The power supply 908 also powers the processing unit 904. Oxygen generated by the oxygen generator/concentrator 810 is pumped through a compression pump 910 and a pressure switch 921 before being delivered to the combination therapy pad 804.

Still referring to FIGURE 9, in a typical embodiment, a water/alcohol reservoir 912 is in fluid communication with a fluid pump 914 and a thermoelectric cooler 916. The thermoelectric cooler 916 is controlled by the processing unit 904. In a typical embodiment, a vacuum pump 918 is powered by the power supply 908. A collection chamber 920 is fluidly connected to the vacuum pump 918 and the pressure switch 921. The pressure switch 921 is fluidly coupled to the combination therapy pad 804. In a typical embodiment, oxygen therapy and vacuum therapy are each administered to the combination therapy pad 804 through a common port 922. In a typical embodiment, the pressure switch 921 is capable of adjusting the combination therapy pad 804 between vacuum treatment and oxygenation therapy.

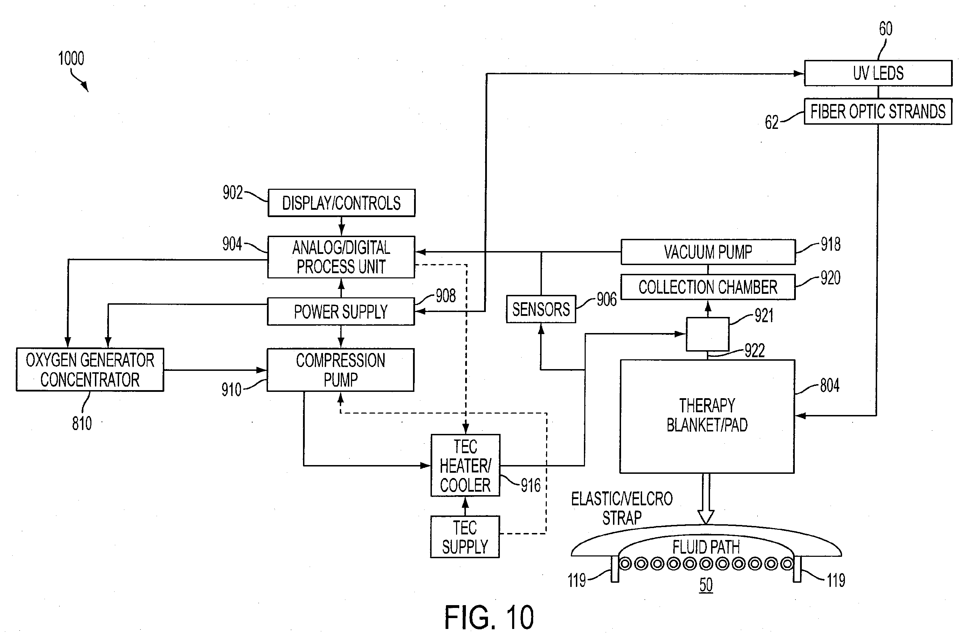

FIGURE 10 is a block diagram of a wound care system according to an exemplary embodiment. In a typical embodiment, a wound care system 1000 is similar in construction to the arrangement described above with respect to FIGURE 9. However, the wound care system 1000 does not include a water/alcohol reservoir or a fluid pump as shown in FIGURE 9. In a typical embodiment, the thermoelectric cooler 916 is in fluid communication with the compression pump 910. Thus, thermal therapy is supplied to the combination therapy pad 804 through heating and cooling of the oxygen supplied by the oxygen generator/concentrator 810.

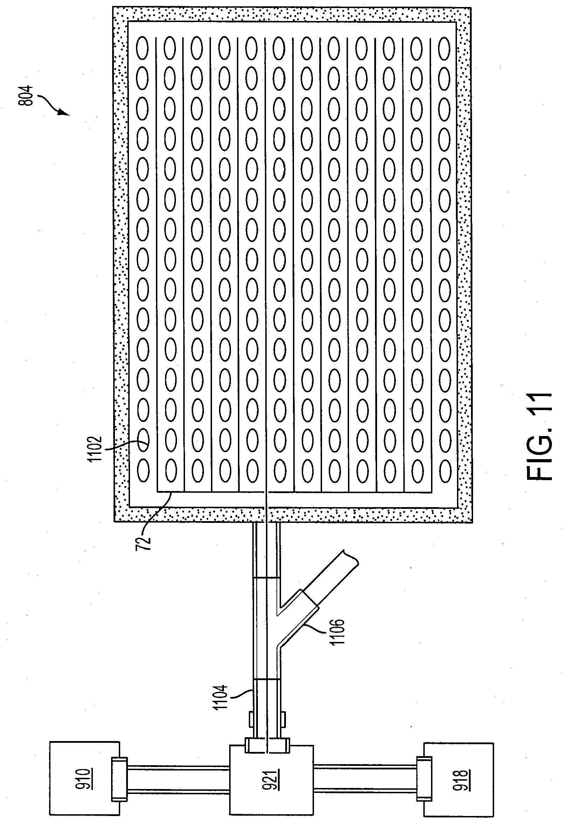

FIGURE 11 is a diagrammatic illustration of a combination therapy pad according to an exemplary embodiment. In a typical embodiment, the combination therapy pad 804 includes a plurality of fiber optic strands 72 to project ultraviolet light onto a wound area (not explicitly shown). In various embodiments, the fiber optic strands 72 may be cleaved or side-emitting fibers; however, one skilled in the art will recognize that any type of fiber-optic strand could be used. In a typical embodiment, the combination therapy pad 804 also includes a plurality of oxygenation/removal ports 1102. In a typical embodiment, the oxygenation/removal ports 1102 alternate between providing oxygen therapy and vacuum therapy to the wound area.

Still referring to FIGURE 11, in a typical embodiment, oxygen therapy and vacuum therapy is administered to the combination therapy pad 804 via an evacuation/oxygenation line 1104. The evacuation/oxygenation line 1104 is fluidly coupled to the pressure switch 921. The pressure switch 921 is fluidly connected to the compression pump 910 and the vacuum pump 918. Thus, in a typical embodiment, the pressure switch 921 is capable of adjusting the combination therapy pad 804 between vacuum treatment and oxygenation therapy.

Still referring to FIGURE 11, in various embodiments, a luer lock 1106 is fluidly coupled to the combination therapy pad 804. During treatment, it is often necessary to administer various medications to a wound site. Such administration often requires removal of a wound dressing such as, for example, the combination therapy pad 804. Frequent removal of the wound dressing can increase risk of further damage to tissue immediately surrounding the wound site. In a typical embodiment, the luer lock 1106 allows for administration of medications and other therapeutic compounds directly to a wound site without the need to remove the combination therapy pad 804.

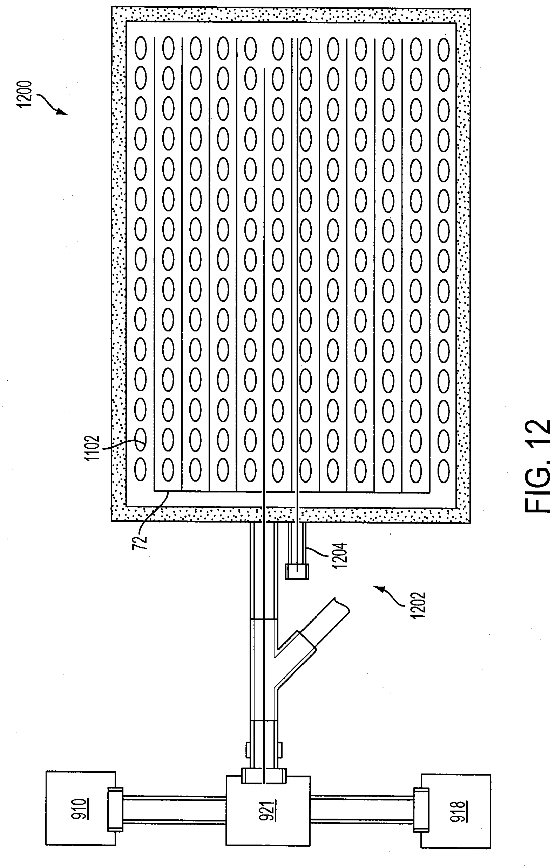

FIGURE 12 is a diagrammatic illustration of a combination therapy pad according to an exemplary embodiment. In a typical embodiment, the combination therapy pad 1200 includes the plurality of fiber optic strands 72 to project ultraviolet light onto a wound area (not explicitly shown). In a typical embodiment, a combination therapy pad 1200 also includes a radio frequency ("RF") antenna 1202. In a typical embodiment, the RF antenna 1202 comprises a wire 1204. The wire 1204 extends along a length of the combination therapy pad 1204. In a typical embodiment, the wire 1204 is disposed within the combination therapy pad 1200 so that, during use, the wire is in close proximity to a wound area. In various embodiments, the wire 1204 is insulated to reduce risk of electric shock to a patient.

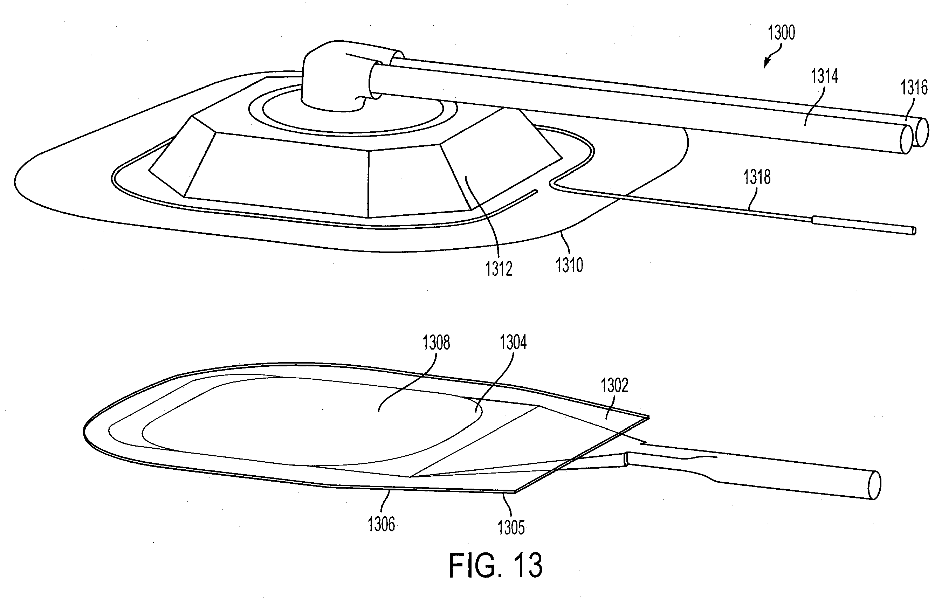

FIGURE 13 is an exploded view of a combination therapy pad according to an exemplary embodiment. A combination therapy pad 1300 includes a first layer 1302 having a first central gap 1304 formed therein. In a typical embodiment, the first layer 1302 is constructed of, for example, urethane. A second layer 1305 is disposed below the first layer 1302 and includes an adhesive bottom surface 1306. A second central gap (not explicitly shown) is formed in the second layer 1305. In a typical embodiment, the second layer 1305 is constructed of, for example, urethane. The first layer 1302 and the second layer 1305 are coupled to each other around a perimeter of the first layer 1302 and the second layer 1305 so that the second central gap aligns with the first central gap 1304. A fiber-optic array 1308 is disposed between the first layer 1302 and the second layer 1305 so as to fill a space defined by the first central gap 1304 and the second central gap.

Still referring to FIGURE 13, a third layer 1310 is disposed above the first layer 1302. The third layer 1310 includes a recessed central area 1312. The recessed central area 1312 is fluidly coupled to a vacuum tube 1314 via a first port and a therapeutic fluid tube 1316 via a second port. An antenna 1318 is coupled to the third layer 1310. The antenna 1318 is formed into a loop and is generally arranged around a perimeter of the recessed central area 1312. In a typical embodiment, the first layer 1302, the second layer 1305, and the third layer 1310 are coupled to each other via a process such as, for example, adhesive bonding or welding.

Still referring to FIGURE 13, during operation, the adhesive bottom surface 1306 is placed on a bodily region of a patient proximate a wound area. In a typical embodiment, the adhesive bottom surface 1306 is oriented such that the second central gap is positioned over the wound area. Thus, the adhesive bottom surface 1306 is not in direct contact with the wound area. The fiber-optic array 1308 is disposed over the wound area and, in various embodiments, may contact the wound area. The fiber-optic array 1308 delivers UV lighting to the wound area thereby promoting cleaning and disinfection of the wound area. The vacuum tube 1314 applies negative pressure to the wound area thereby removing undesirable fluids, tissues, and the like from the wound area. The therapeutic fluid tube 1316 provides a therapeutic fluid such as, for example, oxygen to the wound area. In various embodiments, the therapeutic fluid may be heated or cooled prior to delivery to the wound area. Heating or cooling of the therapeutic fluid allows delivery of thermal therapy to the wound area.

Still referring to FIGURE 13, during operation, a pulsed radio-frequency ("RF") signal having a pulse frequency on the order of, for example 27MHz, is transmitted to the antenna 1318. In a typical embodiment, an amplitude of the pulsed RF signal is on the order of, for example, a fraction of a Watt. Such an amplitude is below a threshold where federal licensing is typically required. The antenna 1318 receives the pulsed RF signal from a radio-frequency source and transmits the pulsed RF signal to a region in close proximity to the wound area. Exposing the wound area to the pulsed RF signal has been shown to be beneficial to healing by encouraging intracellular communication. In particular, pulsed RF signals have been shown to stimulate cellular bonding, and metabolism.

## Claims

1. A combination therapy pad (1300) **characterized in that** it comprises: a first layer (1302);
a second layer (1305) operatively coupled to the first layer (1302);
a fiber-optic array (1308) disposed between the first layer (1302) and the second layer (1305);
a third layer (1310) operatively coupled to the first layer (1302), the third layer (1310) having a vacuum tube (1314) in fluid communication with a vacuum source and a therapeutic fluid tube (1316) in fluid communication with a therapeutic fluid source; and
wherein the third layer (1310) provides at least one of vacuum therapy and therapeutic fluid treatment to a wound area; and
wherein:
the first layer (1302) comprises a first central gap (1304);
the second layer (1305) comprises a second central gap substantially aligned with the first central gap (1304); and
at least a portion of the fiber-optic array (1308) is disposed in the first central gap (1302) and the second central gap.

2. The combination therapy pad (1300) of claim 1, wherein the portion of the fiber-optic array (1308) is placed in direct contact with the wound area.

3. The combination therapy pad (1300) of claim 1, wherein the second layer (1305) comprises an adhesive bottom surface (1306).

4. The combination therapy pad (1300) of claim 1, wherein:
a recessed central area (1312), in fluid communication with the vacuum tube and the therapeutic fluid tube, is defined within the third layer (1310).

5. The combination therapy pad (1300) of claim 4, comprising an antenna (1318) disposed substantially around a perimeter of the recessed central area (1312), wherein the antenna (1318) receives pulsed radio-frequency signals.

6. The combination therapy pad (1300) of claim 1, wherein the first layer (1302), the second layer (1305), and the third layer (1310) are joined via at least one of adhesive bonding and welding.

## Patentansprüche

1. Kombinationstherapiekissen (1300), **dadurch gekennzeichnet, dass** es umfasst:
eine erste Schicht (1302);
eine zweite Schicht (1305), welche zusammenwirkend mit der ersten Schicht (1302) gekoppelt ist;
eine Fieberoptikanordnung (1308), welche zwischen der ersten Schicht (1302) und der zweiten Schicht (1305) angeordnet ist;
eine dritte Schicht (1310), welche zusammenwirkend mit der ersten Schicht (1302) gekoppelt ist, wobei die dritte Schicht (1310) einen Vakuumschlauch (1314), welcher in einer Fluidverbindung mit einer Vakuumquelle steht, und einen Therapiefluidschlauch (1316) aufweist, welcher in Fluidverbindung mit einer therapeutischen Fluidquelle steht; und
wobei die dritte Schicht (1310) zumindest eine von Vakuumtherapie und therapeutische Fluidbehandlung zu einem Wundbereich bereitstellt; und
wobei:
die erste Schicht (1302) zumindest einen ersten zentralen Spalt (1304) umfasst;
die zweite Schicht (1305) einen zweiten zentralen Spalt, welcher im Wesentlichen mit dem ersten zentralen Spalt (1304) ausgerichtet ist, umfasst; und
zumindest ein Abschnitt der ersten Fieberoptikanordnung (1308) im ersten zentralen Spalt (1302) und dem zweiten zentralen Spalt angeordnet ist.

2. Kombinationstherapiekissen (1300) nach Anspruch 1, wobei der Abschnitt der Fieberoptikanordnung (1308) in direktem Kontakt mit der Wundfläche angeordnet ist.

3. Kombinationstherapiekissen (1300) nach Anspruch 1, wobei die zweite Schicht (1305) einen Haftbodenfläche (1306) umfasst.

4. Kombinationstherapiekissen (1300) nach Anspruch 1, wobei:
eine vertiefte zentrale Fläche (1312), welche in Fluidverbindung mit dem Vakuumschlauch und dem Therapiefluidschlauch steht, innerhalb der dritten Schicht (1310) definiert ist.

5. Kombinationstherapiekissen (1300) nach Anspruch 4, umfassend eine Antenne (1318), welche im Wesentlichen um einen Umfang der vertieften zentralen Fläche (1312) angeordnet ist, wobei die Antenne (1318) gepulste Radiofrequenzsignale empfängt.

6. Kombinationstherapiekissen (1300) nach Anspruch 1, wobei die erste Schicht (1302), die zweite Schicht (1305) und die dritte Schicht (1310) mittels zumindest eines von Haftbonden oder Schweißen verbunden sind.

## Revendications

1. Tampon de thérapie de combinaison (1300) **caractérisé en ce qu'**il comprend :
une première couche (1302) ;
une deuxième couche (1305) fonctionnellement couplée à la première couche (1302) ;
un réseau de fibres optiques (1308) disposé entre la première couche (1302) et la deuxième couche (1305) ;
une troisième couche (1310) fonctionnellement couplée à la première couche (1302), la troisième couche (1310) ayant un tube sous vide (1314) en communication fluidique avec une source de vide et un tube de fluide thérapeutique (1316) en communication fluidique avec une source de fluide thérapeutique ; et
dans lequel la troisième couche (1310) apporte au moins l'un parmi une thérapie sous vide et un traitement par fluide thérapeutique à une plaie ; et
dans lequel :
la première couche (1302) comprend un premier espace central (1304) ;
la deuxième couche (1305) comprend un second espace central sensiblement aligné sur le premier espace central (1304) ; et
au moins une partie du réseau de fibres optiques (1308) est disposée dans le premier espace central (1302) et le deuxième espace central.

2. Tampon de thérapie de combinaison (1300) selon la revendication 1, dans lequel la partie du réseau de fibres optiques (1308) est placée en contact direct avec la plaie.

3. Tampon de thérapie de combinaison (1300) selon la revendication 1, dans lequel la deuxième couche (1305) comprend une surface inférieure adhésive (1306).

4. Tampon de thérapie de combinaison (1300) selon la revendication 1, dans lequel :
une zone centrale évidée (1312), en communication fluidique avec le tube sous vide et le tube de fluide thérapeutique, est définie dans la troisième couche (1310).

5. Tampon de thérapie de combinaison (1300) selon la revendication 4, comprenant une antenne (1318) disposée sensiblement autour d'un périmètre de la zone centrale évidée (1312), dans lequel l'antenne (1318) reçoit des signaux de fréquence radio pulsés.

6. Tampon de thérapie de combinaison (1300) selon la revendication 1, dans lequel la première couche (1302), la deuxième couche (1305) et la troisième couche (1310) sont reliées par le biais d'au moins une technique parmi la liaison adhésive et le soudage.
